# EUROPÄISCHE PATENTSCHRIFT

(11) **EP 3 034 123 B1**
(45) Veröffentlichungstag und Bekanntmachung des Hinweises auf die Patenterteilung: **25.04.2018**
(21) Anmeldenummer: 15003473.4
(22) Anmeldetag: 07.12.2015
(51) Int. Cl.: A61M 16/18, A61M 16/10, A61M 16/08

(54) **NARKOSEMITTELVERDUNSTUNGSEINHEIT**
ANESTHESIA AGENT EVAPORATING UNIT
UNITÉ DE VAPORISATION DE PRODUIT ANESTHÉSIQUE

(30) Priorität: 17.12.2014 DE 102014018602
(43) Veröffentlichungstag der Anmeldung: 22.06.2016
(73) Patentinhaber: Drägerwerk AG & Co. KGaA, 23558 Lübeck (DE)
(72) Erfinder: Radomski, Klaus, 23566 Lübeck (DE); Lutter, Thomas, 23558 Lübeck (DE); Mair, Franz, 80997 München (DE); Heidschmidt, Michael, 23558 Lübeck (DE)
(74) Vertreter: Heinemeyer, Karsten

(56) Entgegenhaltungen:
- EP-A1- 0 454 390
- WO-A1-98/44977
- WO-A1-2008/095245
- DE-A1- 3 523 947
- DE-A1- 4 110 211
- DE-B3-102005 005 349
- DE-B3-102007 015 038
- GB-A- 2 279 015

## Beschreibung

Die Erfindung betrifft eine Narkosemittelverdunstungseinheit mit einem Narkosemittelbehälter zur Aufnahme von flüssigem Narkosemittel, einer Gasleitung mit einer offenporigen, porösen Leitungswandung zum Leiten von Gas und zum Anreichern des Gases mit Narkosemittel, wobei die Leitungswandung mit Narkosemittel aus dem Narkosemittelbehälter versorgt wird, und einer Heizung zum Beheizen der Gasleitung.

Narkosemittelverdunstungseinheiten verschiedener Art sind aus dem Stand der Technik grundsätzlich bekannt. Die Narkosemittelverdunstungseinheiten dienen zur Anreicherung eines Gases, das vorzugsweise auch als Trägergas bezeichnet wird, mit einem flüchtigen Narkosemittel. Das flüchtige Narkosemittel wird dazu in einem Narkosemittelbehälter der Narkosemittelverdunstungseinheit gelagert. Um das Narkosemittel an ein Gas, welches durch eine Gasleitung der Narkosemittelverdunstungseinheit strömt, abzugeben, ist die Leitungswandung der Gasleitung offenporig porös. Mittels Kapillarkräfte kann also das flüssige Narkosemittel von der Leitungswandung aufgenommen, gespeichert und abgegeben werden. Die Abgabe erfolgt dabei vorzugsweise in den innenliegenden Kanal, welcher von der Gasleitung gebildet wird. Hier strömt das Gas bzw. das Trägergas, welches sich durch eine Abgabe von dem Narkosemittel aus den Poren der Leitungswandung mit dem, sich bei der Abgabe verflüchtigenden, Narkosemittel anreichert. Die Länge der Gasleitung ist dabei vorzugsweise so gewählt, dass eine gewünschte Anreicherung des Gases mit Narkosemittel erreicht wird. Die Gasleitung mit der zugehörigen, offenporigen, porösen Leitungswandung kann zwar das Narkosemittel aufnehmen, speichern und zur radialen Innenseite hin abgeben, jedoch ist die Gasleitung nicht als primärer Speicher für das flüssige Narkosemittel vorgesehen. Hierzu dient der Narkosemittelbehälter. Um die Gasleitung, bzw. die zugehörige Leitungswandung, immer mit einer ausreichenden Menge von Narkosemittel zu versorgen, ist ein narkosemittelleitender Docht vorgesehen. Bei dem Docht handelt es sich beispielsweise um einen Docht aus einem Gewebematerial. Alternativ oder ergänzend kann ein poröser, vorzugsweise offenporiger, Festkörperdocht vorgesehen sein. Ein derartiger Festkörperdocht ist vorzugsweise aus einem porösen Sintermetall oder aus einer porösen Sinterkeramik. Alternativ kann der Festkörperdocht auch aus einem porösen, offenporigen Kunststoff hergestellt sein. Entsprechendes gilt im Übrigen für die Leitungswandung der Gasleitung. Indem sich der Docht von einem Innenraum des Narkosemittelbehälters zu der Leitungswandung erstreckt, kann das flüssige Narkosemittel mittels Kapillarkräfte von dem Narkosemittelbehälter zu der Leitungswandung transportiert werden. Der Docht dient also zur Versorgung der Leitungswandung mit Narkosemittel aus dem Narkosemittelbehälter.

Um ein durch die Gasleitung strömendes Gas mit Narkosemittel anzureichern, muss das Narkosemittel an der Grenzfläche von der Leitungswandung zum Gas verdunsten. Grundsätzlich ist das Narkosemittel dazu geeignet, da es sich bei dem Narkosemittel vorzugsweise um ein flüchtiges Narkosemittel handelt. Für die Verdunstung des Narkosemittels bedarf es jedoch einer entsprechenden Leistung bzw. Energie. Die für die Verdunstung von dem flüssigen Narkosemittel erforderliche Verdunstungsenergie bzw. Verdunstungsleistung wird bei einigen herkömmlichen Narkosemittelverdunstungseinheiten mittels Wärmeleitung aus einem meist metallischen Teil der Narkosemittelverdunstungseinheit zur Verfügung gestellt. Mit anderen Worten muss für die gewünschte Verdunstung des Narkosemittels ein bestimmter Wärmestrom zur Verfügung gestellt werden, der sich proportional zur spezifischen Wärmekapazität des genannten metallischen Teils, der Masse des metallischen Teils und der Temperaturdifferenz des metallischen Teils bei der Abkühlung verhält. Da die spezifische Wärmekapazität des metallischen Teils konstant ist und für gewöhnlich nur eine begrenzte, oftmals geringe, Temperaturdifferenz erreichbar ist, muss die Masse des metallischen Teils an die erforderliche Verdunstungsleistung bzw. Verdunstungsenergie angepasst werden. Denn andernfalls besteht die Gefahr, dass mit der Verdunstung eine Abkühlung des metallischen Teils einhergeht, wobei ab einer bestimmten Temperaturgrenze des metallischen Teils kein Energietransport zur Verdunstung mehr möglich ist. Um dies zu vermeiden, ist bei konventionellen Narkosemittelverdunstungseinheiten ein derartiges metallisches Teil mit einer Masse von mehreren Kilogramm ausgestaltet, was die Handhabung einer entsprechenden Narkosemittelverdunstungseinheit spürbar erschwert. Außerdem haben die Materialkosten des metallischen Teils einen erheblichen Beitrag.

GB 2 279 015 A beschreibt eine Narkosemittelverdunstungseinheit mit einem Narkosemittelbehälter und einer Gasleitung. Die Gasleitung hat einen offenporigen, porösen Leitungsabschnitt, den das Gas beim Durchleiten passiert. Der poröse Gasleitungsabschnitt steht in Kontakt mit einem bis in das Narkosemittel im Narkosemittelbehälter hineinreichenden Docht, um den porösen Leitungsabschnitt mit flüssigem Narkosemittel zu versorgen. Das Flüssigkeit absorbierende Material im porösen Leitungsabschnitt und der Docht sind als separate Komponenten beschrieben.

Eine sehr ähnliche Narkosemittelverdunstungseinheit ist in DE 35 23 947 A1 beschrieben. Auch hier hat die Gasleitung einen Leitungsabschnitt, in dem sich ein poröses Dochtmaterial befindet. Das Dochtmaterial in dem genannten Leitungsabschnitt steht in Kontakt mit einem separaten Dochtmantel, dessen von dem Material in dem genannten Leitungsabschnitt entferntes Ende bis in das Narkosemittel im Narkosemittelbehälter reicht, um so das Dochtmaterial in dem genannten Leitungsabschnitt über den Dochtmantel mit Narkosemittel zu versorgen.

Aus DE 10 2005 005 349 B3 ist eine weitere Narkosemittelverdunstungseinheit mit einem Narkosemittelbehälter und einer Gasleitung mit einer gasdichten Leitungswandung bekannt. Ein poröser Dochtkörper (Festkörperdocht) reicht aus dem Innenraum des Narkosemittelbehälters bis zur Gasleitung und durch die Leitungswandung hindurch in das Innere der Gasleitung hinein, um das Innere der Gasleitung mit Narkosemittel zu versorgen. Ferner ist eine Heizung vorhanden, die auf den Dochtkörper in einem Bereich außerhalb der Gasleitung vor dessen Eintritt in diese einwirkt.

Aus WO 98/44977 A1 ist eine Narkosemittelverdunstungseinheit mit den Merkmalen des Oberbegriffs von Anspruch 1 bekannt. Die Narkosemittelverdunstungseinheit hat einen Narkosemittelbehälter zur Aufnahme von flüssigem Narkosemittel und eine Gasleitung mit einer offenporigen, porösen Leitungswandung zum Leiten von Gas und zum Anreichern des Gases mit Narkosemittel, wobei die Leitungswandung mit Narkosemittel aus dem Narkosemittelbehälter versorgt wird, und zwar durch eine zwischen dem Narkosemittelbehälter und der Leitungswandung verlaufende Röhre, durch die mittels einer Pumpe Narkosemittel zur Leitungswandung gefördert wird. Ferner ist eine auf die Gasleitung einwirkende Heizung vorhanden, um diese zu beheizen.

Der Erfindung liegt die Aufgabe zugrunde, eine Narkosemittelverdunstungseinheit bereitzustellen, die besonders einfach und günstig herstellbar ist und eine effektive Verdunstung des Narkosemittels bewirkt.

Gelöst wird die Aufgabe durch eine Narkosemittelverdunstungseinheit mit den Merkmalen des Anspruchs 1. Vorgesehen ist also eine Narkosemittelverdunstungseinheit mit einem Narkosemittelbehälter zur Aufnahme von flüssigem Narkosemittel, einer Gasleitung mit einer offenporigen, porösen Leitungswandung zum Leiten von Gas und zum Anreichern des Gases mit Narkosemittel, wobei die Leitungswandung mit Narkosemittel aus dem Narkosemittelbehälter versorgt wird, wobei die Narkosemittelverdunstungseinheit eine Heizung zum Beheizen der Gasleitung aufweist. Erfindungsgemäß ist zur Versorgung der Leitungswandung mit Narkosemittel ein sich aus einem Innenraum des Narkosemittelbehälters zu der Leitungswandung erstreckender Docht vorgesehen. Ferner sind die Gasleitung und der Docht integral ausgestaltet.

Die Gasleitung und der Docht sind also integral ausgestaltet, sie bilden also eine integrale Einheit. Dies erleichtert den Transport des flüssigen Narkosemittels aus dem Innenraum des Narkosemittelbehälters zu der Leitungswandung. Mit anderen Worten ist der Widerstand für den Transport des flüssigen Narkosemittels, der mittels Kapillarkräften aus dem Narkosemittelbehälter zu der radialinnenseitigen Fläche der Leitungswandung gedrückt wird, verringert. Mit dem verringerten Widerstand kann eine höhere Menge an flüssigem Narkosemittel aus dem Innenraum des Narkosemittelbehälters zu der Leitungswandung in vergleichbarer Zeit transportiert werden. Durch die Heizung kann diese höhere Menge an flüssigem Narkosemittel verdunstet und an das durch die Gasleitung strömende Gas abgegeben werden. Denn die Heizleistung der Heizung ist einstellbar bzw. steuerbar. Es findet also kein unbeabsichtigter Abbruch der Verdunstung von dem Narkosemittel statt. Zusammenfassend kann mit der Heizung und der integralen Ausgestaltung von dem Docht und der Gasleitung das Narkosemittel auf einem kürzeren Abschnitt der Gasleitung an das durch die Gasleitung strömende Gas übertragen werden. Damit besteht die Möglichkeit, die Gasleitung zu verkürzen, was wiederum die Kompaktheit und die Handhabbarkeit der Narkosemittelverdunstungseinheit verbessert.

Wie aus den vorherigen Abschnitten zu entnehmen ist, findet der Übergang der flüssigen Phase des Narkosemittels zu der gasförmigen Phase an dem Übergang der Leitungswandung zu dem durch die Gasleitung strömenden Gas statt. Die für die entsprechende Verdunstung notwendige Verdunstungsenergie bzw. -leistung wird durch die Heizung bereitgestellt. Diese dient zum Beheizen der Gasleitung und kann die Energie deshalb unmittelbar an der Stelle zuführen, an der die zuvor erläuterte, entsprechende Verdunstung des Narkosemittels stattfindet. Die für die Verdunstung notwendige Verdunstungsenergie bzw. -leistung ist deshalb nicht einem großen und schweren metallischen Teil zu entnehmen. Denn dies wird durch die Heizung gewährleistet. Ein entsprechendes metallisches Teil kann deshalb deutlich kleiner ausgestaltet sein. Es kann auch möglich sein, auf das metallische Teil vollständig zu verzichten. Um einen Wirkungsgrad der durch die Heizung hinzugefügten Energie und der für die Verdunstung notwendigen Verdunstungsenergie möglichst hoch auszugestalten, ist eine Wärmestrahlungsrichtung der Heizung in Richtung der Gasleitung, bzw. der zugehörigen Leitungswandung, ausgerichtet. Die Heizung kann also eine Vorzugsrichtung aufweist, in der die von der Heizung bereitgestellte Wärme abgestrahlt wird. Diese Vorzugsrichtung zeigt sodann in Richtung der Gasleitung bzw. der Leitungswandung. Eine derartige Heizung kann eine aus dem Stand der Technik bekannte Heizung sein. Insbesondere kann die Heizung eine elektrische Heizung sein. Die Heizung kann deshalb ein besonders geringes Gewicht aufweisen. Darüber hinaus ist die Heizung mit einem besonders kleinen Bauraum herstellbar. Eine Narkosemittelverdunstungseinheit mit der Heizung kann deshalb besonders klein bzw. kompakt und mit einem geringen Gewicht hergestellt sein. Dies erleichtert die Handhabung. Außerdem verringert es die Herstellkosten.

Eine bevorzugte Ausgestaltung der Narkosemittelverdunstungseinheit zeichnet sich dadurch aus, dass die Heizung eine Gasleitungsheizung ist. Die Heizung kann also speziell oder besonders dazu ausgebildet sein, eine Gasleitung, bzw. die zugehörige Leitungswandung, mit Wärme zu versorgen. Dies erhöht den Wirkungsgrad bei der Übertragung der Wärme von der Heizung auf die Gasleitung bzw. auf die zugehörige Leitungswandung. Die Heizung kann somit aufgrund ihrer höheren Effizienz kompakter und ggf. mit einer geringeren Leistung ausgestaltet sein. Dies verringert sowohl die Herstellkosten als auch einen für die Heizung notwendigen Bauraumaufwand in der Narkosemittelverdunstungseinheit, was die Handhabbarkeit weiter verbessert.

Eine weitere vorteilhafte Ausgestaltung der Narkosemittelverdunstungseinheit zeichnet sich dadurch aus, dass die Heizung eine elektrische Heizung ist. Eine elektrische Heizung für die Narkosemittelverdunstungseinheit hat sich in der Praxis als besonders vorteilhaft erwiesen, da diese eine besonders geringe zeitliche Trägheit aufweist. Die Heizung kann somit besonders einfach und schnell gesteuert werden, um immer die jeweils erforderliche Wärme an die Gasleitung bzw. die zugehörige Leitungswandung zu übertragen. Mit anderen Worten kann mit einer elektrischen Heizung besonders einfach erreicht werden, dass nicht zu viel und nicht zu wenig Wärme von der Heizung an die Gasleitung bzw. an die zugehörige Leitungswandung übertragen wird.

Eine weitere vorteilhafte Ausgestaltung der Narkosemittelverdunstungseinheit zeichnet sich dadurch aus, dass die Gasleitung von einem Halter gehalten ist, dem die Heizung zugeordnet ist. Die Gasleitung ist mit einer offenporigen, porösen Leitungswandung ausgestaltet. Die Leitungswandung kann also durch ein Textil gebildet sein. Alternativ ist es möglich, dass die Leitungswandung von einem offenporigen, porösen Metall, Kunststoff oder Keramikwerkstoff gebildet ist. Allen Ausgestaltungen der Leitungswandung ist gemein, dass sie oftmals eine geringe Formstabilität aufweisen. Um die Gasleitung an dem gewünschten Ort in der Narkosemittelverdunstungseinheit zu platzieren, kann der Halter vorgesehen sein, der zum Halten der Gasleitung ausgestaltet ist. Der Halter kann also unmittelbaren Kontakt zu der Gasleitung aufweisen. Indem die Heizung dem Halter zugeordnet ist, kann eine besonders dichte Anordnung der Heizung zu der Gasleitung, bzw. der zugehörigen Leitungswandung, gewährleistet werden. Insbesondere kann die Heizung einen Teil des Halters bilden, sodass kein hoher, oder zumindest im Wesentlichen kein zusätzlicher, Bauraum beansprucht wird. Grundsätzlich, aber insbesondere mit Bezug auf den Halter, ist es von Vorteil, wenn die Heizung unmittelbaren Kontakt zu der Gasleitung, bzw. der Leitungswandung, hat. Für den Fall, dass die Heizung einen Teil des Halters bildet, kann der Kontakt zwischen der Heizung und der Gasleitung, bzw. der zugehörigen Leitungswandung, besonders einfach und sicher hergestellt werden. Dabei kann die Heizung korrespondierend zu einer Außenkontur der Leitungswandung ausgebildet sein. Außerdem hat es sich als vorteilhaft erwiesen, wenn der Halter zur Wärmeleitung ausgestaltet ist. So kann der Halter beispielsweise metallisch sein. Der Halter kann somit zur Verteilung der von der Heizung abgegebenen Wärme verwendet werden, um die Leitungswandung über einen möglichst großen Abschnitt mit der Wärme der Heizung zu versorgen. Die Wärme wird also nicht nur punktuell oder über einen kleinen Flächenabschnitt an die Leitungswandung übertragen, sondern kann mittels der besonders guten Wärmeleitungsfähigkeit des Halters über eine große Fläche an die Leitungswandung übertragen werden. Dies verbessert die Verdunstung des Narkosemittels, sodass die Länge der Gasleitung besonders kurz gehalten werden kann. Mit der besonders kurzen Gasleitungslänge kann die Kompaktheit der Narkosemittelverdunstungseinheit weiter verbessert werden.

Eine weitere vorteilhafte Ausgestaltung der Narkosemittelverdunstungseinheit zeichnet sich dadurch aus, dass die Heizung in die Leitungswandung zumindest teilweise integriert ist. Damit kann die Heizung noch dichter an die Stelle platziert werden, an der der Phasenübergang von dem flüssigen Narkosemittel zu dem gasförmigen Narkosemittel stattfindet. Denn mit der Integration der Heizung in die Leitungswandung ist die Heizung nur noch wenig von der radial innenseitigen Wandungsfläche der Leitungswandung entfernt, an der die Verdunstung zumindest im Wesentlichen stattfindet. Mit der Integration der Heizung in die Leitungswandung kann also die Wärmezufuhr gezielt eingebracht werden. Dies erleichtert die Steuerbarkeit der für die Verdunstung erforderliche Leistung bzw. erforderlichen Energie. Mit der Integration der Heizung kann darüber hinaus die Formstabilität der Gasleitung erhöht werden, sodass ein kleinerer Halter zum Halten der Gasleitung verwendet werden kann. Insbesondere kann bei einer besonders formstabilen Ausgestaltung der Heizung auf den Halter vollständig verzichtet werden. Mit der Integration der Heizung in die Leitungswandung kann deshalb der Bauraum reduziert werden, was die Kompaktheit der Narkosemittelverdunstungseinheit weiter erhöht. Für den Fall, dass kein Halter notwendig ist, sinkt darüber hinaus auch das Gewicht der Narkosemittelverdunstungseinheit spürbar, sodass zudem die Handhabbarkeit der Narkosemittelverdunstungseinheit verbessert wird.

Eine weitere vorteilhafte Ausgestaltung der Narkosemittelverdunstungseinheit zeichnet sich dadurch aus, dass die Heizung Lamellen aufweist, die außen benachbart in Kontakt mit der Leitungswandung der Gasleitung angeordnet sind. In der Praxis hat es sich gezeigt, dass für bestimmte Narkosemittel eine Heizung mit einer höheren Wärmeleistung notwendig ist. Um diese Wärmeleistung an die Gasleitung, bzw. an die zugehörige Leitungswandung, zu übertragen, hat es sich als vorteilhaft erwiesen, die zuvor genannten Lamellen für die Heizung vorzusehen. Derartige Lamellen sind zur Wärmeübertragung ausgestaltet. Sie können also die von einer Heizquelle der Heizung abzugebende Wärme an die Gasleitung bzw. die zugehörige Gasleitungswandung übertragen. Durch ihren unmittelbaren Kontakt zu der Leitungswandung wird darüber hinaus ein Wärmeverlust verringert oder vermieden. Mit dem besonders geringen Wärmeverlust lässt sich die Wärmezufuhr mittels der Heizung an die Leitungswandung besonders präzise steuern. Ist die Gasleitung in Schlaufen und/oder Ringen angeordnet, können die Lamellen zwischen zwei benachbarte Abschnitte der Gasleitung fassen, sodass eine Baueinheit aus der Gasleitung und der Heizung besonders kompakt ausgestalte werden kann. Dies verringert das notwendige Bauraumvolumen der Narkosemittelverdunstungseinheit, was die Handhabbarkeit der Narkosemittelverdunstungseinheit verbessert.

Eine weitere vorteilhafte Ausgestaltung der Narkosemittelverdunstungseinheit zeichnet sich dadurch aus, dass der Docht als Textil-Docht und die Gasleitung als Textil-Gasleitung ausgestaltet sind. Das zugehörige Textil kann eine Vielzahl von Fasern aufweisen, um den Docht zu bilden. Zwischen den Fasern können sich von den Fasern begrenzte Räume oder Hohlkammern ausbilden, die aufgrund ihrer zumeist geringen Größe als Poren bezeichnet werden können. Die Poren können flüssiges Narkosemittel aufnehmen. Außerdem können die Poren können untereinander in Fluidverbindung sein, um einen Austausch und/oder Transport von Narkosemittel zu ermöglichen. Außenseitig sind die Poren von den Fasern nicht geschlossen. Es kann deshalb auch von offenen Poren gesprochen werden. Mit anderen Worten kann der Textil-Docht außenseitig offenporig ausgestaltet sein. Bezüglich der vorteilhaften Merkmale und Effekte des Textils für die Gasleitung wird auf die vorherige Erläuterung analog Bezug genommen. Somit kann das Textil für die Gasleitung ebenfalls Fasern aufweisen, die Poren zur Aufnahme von flüssigen Narkosemittel ausbilden. Mit den Fasern bzw. den Poren ist die Textil-Gasleitung deshalb zum Transport von flüssigen Narkosemittel ausgestaltet. Außerdem können die Fasern bzw. die Poren derart ausgestaltet sein, um an der Außenseite der Gasleitung zumindest abschnittweise offenporig zu sein, um einen Übergang des Narkosemittels an das Trägergas zu ermöglichen.
Eine weitere vorteilhafte Ausgestaltung der Narkosemittelverdunstungseinheit zeichnet sich dadurch aus, dass ein Temperatursensor zur Erfassung von einer Temperatur des Gases in der Gasleitung vorgesehen ist. Anhand der Temperatur des durch die Gasleitung strömenden Gases kann die Sättigungskonzentration mit Narkosemittel bestimmt werden. Der Temperatursensor kann deshalb vorteilhaft zur Steuerung der Narkosemittelverdunstungseinheit, und insbesondere der Heizung, verwendet werden. Wird beispielsweise eine höhere Konzentration an Narkosemittel in dem durch die Gasleitung strömenden Gas gewünscht, kann der Temperatursensor darüber Aufschluss geben, ob diese Konzentration grundsätzlich erreichbar ist und falls ja, wie die Heizung zu steuern ist, um eine entsprechende Verdunstung des Narkosemittels und damit einer Übertragung des Narkosemittels an das Gas zu gewährleisten.

Eine weitere vorteilhafte Ausgestaltung der Narkosemittelverdunstungseinheit zeichnet sich dadurch aus, dass ein Temperatursensor zur Erfassung einer die Temperatur des durch die Gasleitung strömenden Gases repräsentierenden Temperatur vorgesehen ist. So kann der Temperatursensor beispielsweise die Temperatur der Leitungswandung, des Halters und/oder eines anderen benachbarten Mittels messen, um anhand dieser gemessenen Temperatur auf die Temperatur des Gases, zumindest annäherungsweise oder zumindest im Wesentlichen, zu schließen. Mit dieser die Gastemperatur repräsentierenden Temperatur kann analog zu der gemessenen Gastemperatur eine verbesserte Steuerung der Heizung und/oder andere Auswertungen, wie sie zuvor erläutert wurden, vorgenommen werden. Dies verbessert die Qualität der Anreicherung des durch die Gasleitung strömenden Gases mit Narkosemittel, bzw. es lässt sich eine genauere Konzentration des Narkosemittels in dem durch die Gasleitung strömenden Gases einstellen.

Gemäß einem weiteren Aspekt wird die eingangs genannte Aufgabe gelöst durch ein Verfahren zum Kontrollieren der erfindungsgemäßen Narkosemittelverdunstungseinheit in der Ausgestaltung, in der ein Temperatursensor zur Erfassung von einer Temperatur des Gases in der Gasleitung und/oder einer diese repräsentierenden Temperatur vorgesehen ist, wobei die Leistung der Heizung in Abhängigkeit von einem Gasstrom durch die Gasleitung, der mittels des Temperatursensors erfassten Temperatur und/oder einer Narkosemittelkonzentration des durch die Gasleitung strömenden Gases kontrolliert wird. Diesem Aspekt der Erfindung liegt der Gedanke zugrunde, dass bei einem höheren Gasstrom die Gasleitung bzw. die Leitungswandung ebenfalls mit einer höheren Menge an Narkosemittel pro Zeiteinheit versorgt werden muss, um die gewünschte Narkosemittelkonzentration in dem durch die Gasleitung strömenden Gas zu erreichen. Dazu kann für die Narkosemittelverdunstungseinheit ein Strömungssensor vorgesehen sein, der die Gasströmung von einem Gas durch die Gasleitung erfassen kann. Hierbei kann es sich um einen Volumenstromsensor und/oder um einen Massenstromsensor handeln. Sensoren, die die zuvor genannten Werte repräsentierenden Werte messen, sollen dabei mit erfasst sein. Die Temperatur des durch die Gasleitung strömenden Gases ist darüber hinaus ausschlaggebend für die maximale Konzentration an Narkosemittel in dem durch die Gasleitung strömenden Gas. Mit dieser Temperatur kann also ermittelt werden, wie viel Narkosemittel durch Verdunstung von dem flüssigen Narkosemittel in der Leitungswandung an das durch die Gasleitung strömende Gas übertragbar ist. Außerdem kann die Heizleistung mit der gemessenen Temperatur des Gases limitiert sein, um einen Niederschlag des Narkosemittels in einem Kanalbereich zu verhindern, der an einen Ausgang der Gasleitung anschließt. Mit dem Erfassen der Temperatur des Gases in der Gasleitung kann also ein besonders sicherer Betrieb der Narkosemittelverdunstungseinheit gewährleistet werden. Mit der Erfassung der Narkosemittelkonzentration in dem durch die Gasleitung strömenden Gas, insbesondere an einem Ausgang der Gasleitung, kann eine sichere Aussage darüber gewährleistet werden, ob die gewünschte Narkosemittelkonzentration erreicht ist oder nicht. Sollte die Narkosemittelkonzentration nicht erreicht sein, kann für die Narkosemittelverdunstungseinheit eine entsprechende Regelung vorgesehen sein, um die Verdunstung des Narkosemittels zu erhöhen oder zu verringern, indem die Regelung die Heizleistung der Heizung erhöht bzw. verringert. Mit anderen Worten kann durch die Erfassung der Narkosemittelkonzentration und einem Vergleich mit einer gewünschten Narkosemittelkonzentration die Heizleistung der Heizung geregelt werden. Mit einer derartigen Ausgestaltung kann die Narkosemittelverdunstungseinheit immer die gewünschte Narkosemittelkonzentration des aus der Gasleitung ausströmenden Gases bereitstellen, was eine besonders sichere Narkotisierung erlaubt.

Die Erfindung wird nachstehend anhand von Ausführungsbeispielen unter Bezugnahme auf die Zeichnungen näher erläutert. In den Zeichnungen zeigen:
- Fig. 1: eine erste, schematische Schnittdarstellung der Narkosemittelverdunstungseinheit,
- Fig. 2: eine zweite, schematische Schnittdarstellung der Narkosemittelverdunstungseinheit, und
- Fig. 3: eine dritte, schematische Querschnittsdarstellung der Narkosemittelverdunstungseinheit.

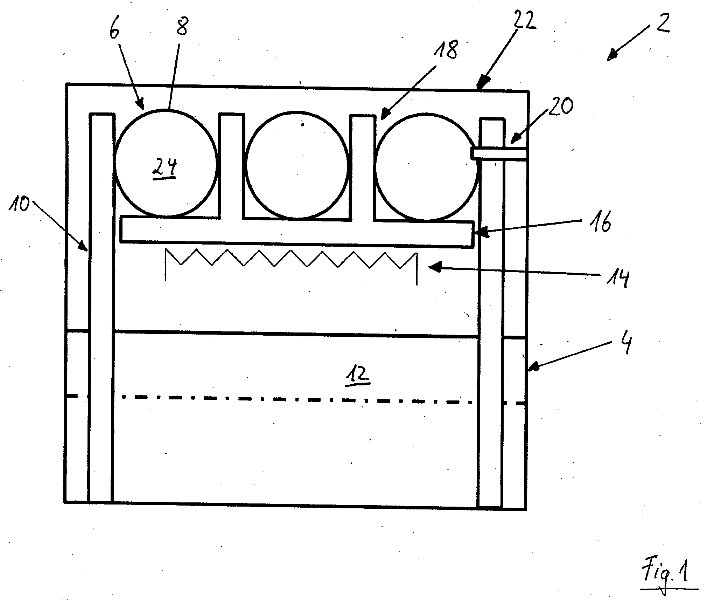

Aus der Figur 1 ist die erfindungsgemäße Narkosemittelverdunstungseinheit 2 schematisch in einer Querschnittansicht zu erkennen. An ihrem Boden ist der Narkosemittelbehälter 4 angeordnet. Der Narkosemittelbehälter 4 dient zur Aufnahme von flüssigem Narkosemittel. Dazu kann ein Verschluss vorgesehen sein, mit dem das flüssige Narkosemittel in den Innenraum 12 des Narkosemittelbehälters 4 einfüllbar ist. Wie aus der Figur 1 durch die gestrichelte Linie angedeutet ist, ist der in Figur 1 gezeigte Narkosemittelbehälter 4 etwa zur Hälfte mit flüssigem Narkosemittel gefüllt. Oberhalb des Narkosemittelbehälters 4 schließt die Verdunstungsbaugruppe 22 der Narkosemittelverdunstungseinheit 2 an. Die Verdunstungsbaugruppe 22 umfasst ein Gehäuse, das mit dem Narkosemittelbehälter 4 verbunden und/oder mit diesem integral ausgestaltet ist. Durch oder in dem Gehäuse der Verdunstungsbaugruppe 22 führt die Gasleitung 6, die zum Leiten von Gas ausgebildet ist. An einem Eingang der Gasleitung 6 strömt Trägergas in die Gasleitung 6 ein, um sodann während des Durchströmens der Gasleitung 6 mit Narkosemittel angereichert zu werden. Dazu weist die Gasleitung 6 eine offenporige, poröse Leitungswandung 8 auf. Am Ende der Gasleitung 6 strömt deshalb das mit Narkosemittel angereicherte Trägergas aus der Gasleitung 6 aus. Dieses mit Narkosemittel angereicherte Trägergas kann sodann zur Narkotisierung einer Person verwendet werden. Um das Narkosemittel aus dem Narkosemittelbehälter 4 zu der Gasleitung 6 zu transportieren, ist ein Docht 10 vorsehen. Der Docht 10 ist zur Leitung bzw. zum Transport von flüssigem Narkosemittel ausgebildet. Ein derartiger Docht 10 ist beispielsweise als ein Textildocht oder als ein poröser, zumindest abschnittsweise offenporiger Kunststoff, Keramikwerkstoff oder Metallwerkstoff-Docht ausgestaltet. Der Docht 10 eignet sich also zum Leiten von Narkosemittel mittels Kapillarkraft aus dem Innenraum 12 des Narkosemittelbehälters 4 zu der Gasleitung 6. Dazu erstreckt sich der Docht 10 entsprechend, nämlich von dem Innenraum 12 des Narkosemittelbehälters 4 zu der Gasleitung 6, mit der der Docht 10 als integrales Bauteil ausgestaltet ist. Durch den Docht 10 ist die Leitungswandung 8 der Gasleitung 6 also ausreichend mit flüssigem Narkosemittel versorgbar. Strömt nun Trägergas durch die Gasleitung 6, verdunstet Narkosemittel aus den Poren der Leitungswandung 8 in den umschlossenen Kanalraum 24 der Gasleitung 6, sodass das durch die Gasleitung 6 strömende Gas mit Narkosemittel angereichert wird. Um eine kontinuierliche Verdunstung während eines Strömens von Gas durch die Gasleitung 6 zu gewährleisten, ist die Gasleitung 6 mit Energie, vorzugsweise mit Wärmeenergie, zu versorgen. Denn anderenfalls würde sich die Verdunstung verschlechtern, sodass eine geringere Menge an Narkosemittel an das durchströmende Gas übertragen wird, was zu vermeiden ist. Um die gewünschte oder erforderliche Menge an Narkosemittel von der Leitungswandung 8 der Gasleitung 6 an das die Gasleitung 6 durchströmende Gas zu übertragen, ist die Heizung 14 vorgesehen, die zur Beheizung der Gasleitung 6 ausgestaltet ist.

Wie aus der Figur 1 zu erkennen ist, ist die Heizung 14 unmittelbar benachbart zu, und vorzugsweise in Kontakt mit, einem Halter 16, der zum Halten der Gasleitung 6 ausgestaltet ist. Der Halter 16 hat einen hohen Wärmeleitkoeffizienten, sodass dieser die von der Heizung 14 abgegebene Wärme aufnehmen und besonders gut an die Gasleitung 6 abgeben kann. Dazu kann der Halter 16 in unmittelbarem Kontakt mit der Gasleitung 6 sein. Dies gewährleistet eine besonders gute Wärmeübertragung von der Heizung 14 über den Halter 16 an die Gasleitung 6. Zusammenfassend kann also festgehalten werden, dass die Heizung 14 als Gasleitungsheizung ausgestaltet ist, da vorzugsweise ein unmittelbarer Kontakt zwischen der Heizung 14 und der Gasleitung 6 besteht und/oder ein wärmeleitender Halter 16 dazwischen zur Wärmeübertragung und/oder -verteilung vorgesehen ist.

Wie aus der Figur 1 zu entnehmen ist, kann die Gasleitung 6 abschnittsweise nebeneinander angeordnet sein. Dies ist beispielsweise dann der Fall, wenn die Gasleitung 6 schlangenförmig oder spiralförmig angeordnet ist. In diesem Fall hat es sich als vorteilhaft erwiesen, wenn die Heizung 14 und/oder der Halter 16 Lamellen 18 aufweist bzw. weisen, die zwischen Leitungsabschnitte der Gasleitung 6 fassen. Die Lamellen 18 sind deshalb von außen benachbart in Kontakt mit der Gasleitungswandung 8 der Gasleitung 6. Dies gewährleistet eine besonders gute Wärmeübertragung der von der Heizung 14 abgegebenen Wärme an die Leitungswandung 8 der Gasleitung 6.

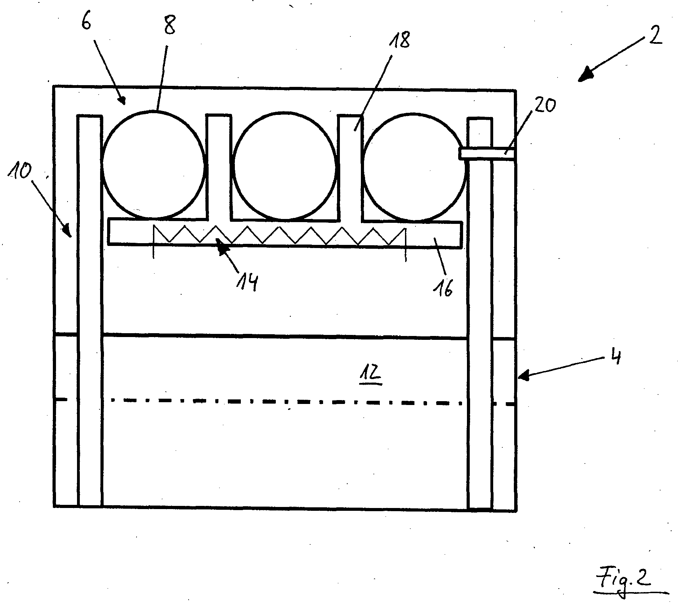

Aus der Figur 2 ist eine weitere Ausgestaltung der erfindungsgemäßen Narkosemittelverdunstungseinheit 2 zu erkennen. In ihrem grundlegenden Aufbau gleicht die Narkosemittelverdunstungseinheit 2 aus Figur 2 der Narkosemittelverdunstungseinheit 2 aus Figur 1. Es wird deshalb in analoger Weise auf die vorangegangenen Erläuterungen Bezug genommen, soweit es sinnvoll ist. Ein Unterschied dieser Ausgestaltung der Narkosemittelverdunstungseinheit 2 aus Figur 2 ist in der Anordnung der Heizung 14 zu sehen. Die Heizung 14 ist dem Halter 16 zugeordnet. Dabei ist die Heizung 14 in den Halter 16 integriert. Der Halter 16 ist vorzugsweise als metallischer Halter 16 ausgestaltet. Somit hat der Halter 16 einen hohen Wärmeleitkoeffizienten und ist besonders gut zum Transport und der Verteilung der von der Heizung 14 abgegebenen Wärme an die Leitungswandung 8 der Gasleitung 6 geeignet. Die Lamellen 18 sind dabei ebenfalls von dem Halter 16 ausgebildet, sodass die Leitungswandung 8 der Gasleitung 6 an zumindest mehreren Stellen über den Ringumfang von dem Halter 16 kontaktiert ist, sodass eine möglichst verteilte Wärmezufuhr gewährleistet wird. Das von der Leitungswandung 8 der Gasleitung 6 aufgenommene, flüssige Narkosemittel, was sich in den Poren befindet, kann durch die Wärmezufuhr erwärmt werden, sodass eine Verdunstung und somit eine Übertragung des Narkosemittels auf das durch die Gasleitung 6 strömende Gas erleichtert ist. Außerdem kann durch die kontinuierliche Wärmezufuhr mittels der Heizung 14 gewährleistet werden, dass auch die Übertragung des Narkosemittels aus den Poren der Leitungswandung 8 zu dem durch die Gasleitung 6 strömenden Gases kontinuierlich, und vorzugsweise wie gewünscht, erfolgt.

Um am Ausgang der Gasleitung 6 das durch die Gasleitung 6 strömende Gas mit der gewünschten Menge an Narkosemittel anzureichern, bzw. um die gewünschte Narkosemittelkonzentration, zu erreichen, kann ein Temperatursensor 20 vorgesehen sein. Der Temperatursensor 20 ist vorzugsweise an einem Ausgangsabschnitt der Gasleitung 6 angeordnet.

Alternativ kann der Temperatursensor 20 auch an einem anderen Abschnitt der Gasleitung 6 angeordnet sein. Mit dem Temperatursensor 20 kann die Temperatur des durch die Gasleitung 6 strömenden Gases erfasst werden. Dazu kann der Temperatursensor 20 in die Leitungswandung 8 integriert sein und/oder in den von der Gasleitung 6 gebildeten Kanalraum 24 hineinragen. Mit der erfassten Temperatur des durch die Gasleitung 6 strömenden Gases kann bestimmt werden, wie viel Narkosemittel dem durch die Gasleitung 6 strömenden Gas zugeführt werden kann. Mit anderen Worten lässt sich die maximale Narkosemittelkonzentration bestimmen. Korrespondierend kann in einem erfindungsgemäßen Verfahren, sofern die maximale Narkosemittelkonzentration in dem durch die Gasleitung 6 strömenden Gas noch nicht erreicht ist, die Temperatur der Heizung 14 erhöht werden, um die jeweils aktuelle Narkosemittelkonzentration in dem durch die Gasleitung 6 strömenden Gas zu erhöhen. Alternativ oder ergänzend kann der Gasstrom durch die Gasleitung 6 erfasst werden, um die Wärmeleistung der mit einem Temperatursensor versehenen Narkosemittelverdunstungseinheit in einem erfindungsgemäßen Verfahren zu steuern. In der Praxis hat es sich als vorteilhaft erwiesen, wenn darüber hinaus eine Regelung vorgesehen ist, mit der die Narkosemittelkonzentration des aus der Gasleitung 6 ausströmenden Gases regelbar ist. Somit kann darüber hinaus ein Sensor zur Erfassung der Narkosemittelkonzentration des aus der Gasleitung 6 ausströmenden Gases vorgesehen sein, wobei der erfasste Wert verwendet wird, um die Leistung der Heizung 14 einzustellen. Hierbei können übliche Regelalgorithmen berücksichtigt und/oder verwendet werden.

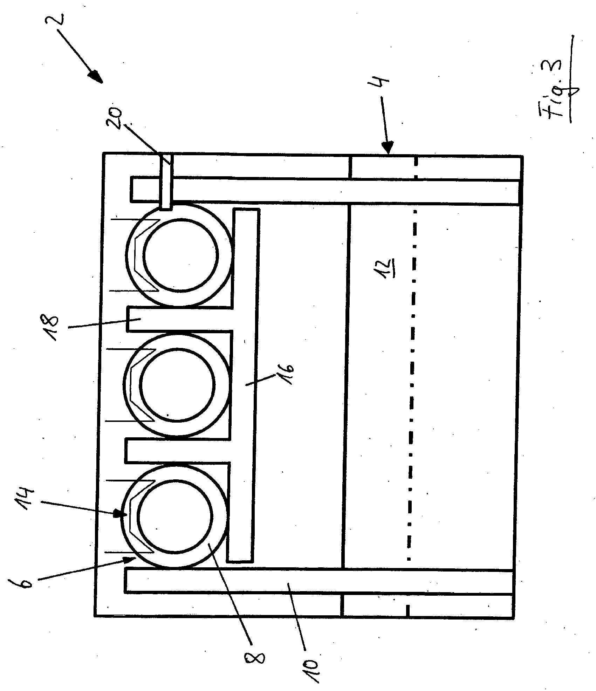

Aus der Figur 3 ist eine weitere Ausgestaltung der erfindungsgemäßen Narkosemittelverdunstungseinheit 2 zu erkennen. Auch hier wird, soweit es sinnvoll ist, auf die vorherigen Erläuterungen zu den Figuren 1 und 2 Bezug genommen, wobei sich die Narkosemittelverdunstungseinheit 2 aus der Figur 3 durch die Anordnung der Heizung 14 unterscheidet. Denn die Heizung 14 ist für diese Ausgestaltung in die Leitungswandung 8 der Gasleitung 6 integriert. Bei der Heizung 14 kann es sich beispielsweise um eine elektrische Drahtheizung handeln. Der elektrische Draht der Heizung 14 kann dazu schlangenförmig, ringförmig oder in einer anderen geeigneten Art in die Leitungswandung 8 der Gasleitung 6 eingebracht sein. Diese Ausgestaltung hat sich als besonders energieeffizient erwiesen, da die von der Heizung 14 hervorgebrachte Wärme unmittelbar an die Gasleitung 6 bzw. an das in den Poren der Leitungswandung 8 befindliche Narkosemittel übertragen wird. Damit finden annähernd keine Wärmeübertragungsverluste statt. Außerdem ist eine derartige Ausgestaltung besonders gut steuerbar, da es annähernd keine zeitliche Verzögerung zwischen dem Zeitpunkt der Abgabe der Wärme von der Heizung 14 und der Aufnahme der entsprechenden Wärme von der Leitungswandung 8 bzw. dem von der Leitungswandung 8 aufgenommenen Narkosemittel gibt. Mit entsprechend kurzen Verzögerungszeiten lässt sich deshalb die Narkosemittelverdunstungseinheit 2 besonders präzise und dynamisch steuern bzw. regeln. Dies unterstützt einen bevorzugt narkosemittelminimalen Betrieb der Narkosemittelverdunstungseinheit 2.

### Bezugszeichenliste

- 2: Narkosemittelverdunstungseinheit
- 4: Narkosemittelbehälter
- 6: Gasleitung
- 8: Leitungswandung
- 10: Docht
- 12: Innenraum
- 14: Heizung
- 16: Halter
- 18: Lamellen
- 20: Temperatursensor
- 22: Verdunstungsbaugruppe
- 24: Kanalraum

## Patentansprüche

1. Narkosemittelverdunstungseinheit (2) mit
- einem Narkosemittelbehälter (4) zur Aufnahme von flüssigem Narkosemittel,
- einer Gasleitung (6) mit einer offenporigen, porösen Leitungswandung (8) zum Leiten von Gas und zum Anreichern des Gases mit Narkosemittel, wobei die Leitungswandung (8) mit Narkosemittel aus dem Narkosemittelbehälter (4) versorgt wird, und
- einer Heizung (14) zum Beheizen der Gasleitung (6),
**dadurch gekennzeichnet, dass** die Leitungswandung (8) durch einen narkosemittelleitenden Docht (10), der sich von einem Innenraum (12) des Narkosemittelbehälters (4) zu der Leitungswandung (8) erstreckt, mit Narkosemittel aus dem Narkosemittelbehälter (4) versorgt wird und dass die Gasleitung (6) und der Docht (10) integral ausgestaltet sind.

2. Narkosemittelverdunstungseinheit (2) nach dem vorhergehenden Anspruch, **dadurch gekennzeichnet, dass** die Heizung (14) eine Gasleitungsheizung ist.

3. Narkosemittelverdunstungseinheit (2) nach einem der vorhergehenden Ansprüche, **dadurch gekennzeichnet, dass** die Heizung (14) eine elektrische Heizung ist.

4. Narkosemittelverdunstungseinheit (2) nach einem der vorhergehenden Ansprüche, **dadurch gekennzeichnet, dass** die Gasleitung (6) von einem Halter (16) gehalten ist, dem die Heizung (14) zugeordnet ist.

5. Narkosemittelverdunstungseinheit (2) nach einem der vorhergehenden Ansprüche, **dadurch gekennzeichnet, dass** die Heizung (14) in die Leitungswandung (8) zumindest teilweise integriert ist.

6. Narkosemittelverdunstungseinheit (2) nach einem der vorhergehenden Ansprüche, **dadurch gekennzeichnet, dass** die Heizung (14) Lamellen (18) aufweist, die außen benachbart in Kontakt mit der Gasleitungswandung (8) der Gasleitung (6) angeordnet sind.

7. Narkosemittelverdunstungseinheit (2) nach einem der vorhergehenden Ansprüche, **dadurch gekennzeichnet, dass** der Docht (10) als Textil-Docht ausgestaltet ist, und dass die Gasleitung (6) als Textil-Gasleitung ausgestaltet ist.

8. Narkosemittelverdunstungseinheit (2) nach einem der vorhergehenden Ansprüche, **dadurch gekennzeichnet, dass** ein Temperatursensor (20) zur Erfassung einer Temperatur des Gases in der Gasleitung (6) und/oder einer diese repräsentierenden Temperatur vorgesehen ist.

9. Verfahren zum Kontrollieren einer Narkosemittelverdunstungseinheit (2) nach Anspruch 8, wobei die Leistung der Heizung (14) in Abhängigkeit von einem Gasstrom durch die Gasleitung (6), der erfassten Temperatur des Gases in der Gasleitung und/oder einer Narkosemittelkonzentration des durch die Gasleitung (6) strömenden Gases kontrolliert wird.

## Claims

1. An anaesthetic evaporation unit (2) having
- an anaesthetic container (4) for receiving liquid anaesthetic,
- a gas line (6) having an open-pore, porous line wall (8) for guiding gas and for enriching the gas with anaesthetic, wherein the line wall (8) is supplied with anaesthetic from the anaesthetic container (4), and
- a heater (14) for heating the gas line (6),
**characterised in that** the line wall (8) is supplied with anaesthetic from the anaesthetic container (4) by means of an anaesthetic-guiding wick (10) that extends from an interior space (12) of the anaesthetic container (4) to the line wall (8), and the gas line (6) and the wick (10) are formed so as to be integral.

2. An anaesthetic evaporation unit (2) according to the preceding claim, **characterised in that** the heater (14) is a gas-line heater.

3. An anaesthetic evaporation unit (2) according to one of the preceding claims, **characterised in that** the heater (14) is an electric heater.

4. An anaesthetic evaporation unit (2) according to one of the preceding claims, **characterised in that** the gas line (6) is held by a holder (16) with which the heater (14) is associated.

5. An anaesthetic evaporation unit (2) according to one of the preceding claims, **characterised in that** the heater (14) is at least partly integrated in the line wall (8).

6. An anaesthetic evaporation unit (2) according to one of the preceding claims, **characterised in that** the heater (14) has ribs (18) which are arranged adjacently to and in contact with the outside of the gas-line wall (8) of the gas line (6).

7. An anaesthetic evaporation unit (2) according to one of the preceding claims, **characterised in that** the wick (10) is formed as a textile wick, and the gas line (6) is formed as a textile gas line.

8. An anaesthetic evaporation unit (2) according to one of the preceding claims, **characterised in that** a temperature sensor (20) is provided to detect a temperature of the gas in the gas line (6) and/or a temperature representing this.

9. A method for controlling an anaesthetic evaporation unit (2) according to claim 8, wherein the output of the heater (14) is controlled as a function of a gas flow through the gas line (6), the detected temperature of the gas in the gas line and/or a concentration of anaesthetic in the gas flowing through the gas line (6).

## Revendications

1. Unité de vaporisation de produit anesthésique (2) avec
- un réservoir de produit anesthésique (4) destiné à contenir un produit anesthésique liquide,
- une conduite de gaz (6) avec une paroi de conduite poreuse à pores ouverts (8) pour conduire du gaz et pour enrichir le gaz en produit anesthésique, dans laquelle la paroi de conduite (8) est alimentée en produit anesthésique à partir du réservoir de produit anesthésique (4), et
- un chauffage (14) pour chauffer la conduite de gaz (6),
**caractérisée en ce que** la paroi de conduite (8) est alimentée en produit anesthésique à partir du réservoir de produit anesthésique (4) au moyen d'une mèche (10) conduisant le produit anesthésique, qui s'étend depuis un espace intérieur (12) du réservoir de produit anesthésique (4) jusqu'à la paroi de conduite (8), et **en ce que** la conduite de gaz (6) et la mèche (10) sont réalisées de façon intégrée.

2. Unité de vaporisation de produit anesthésique (2) selon la revendication précédente, **caractérisée en ce que** le chauffage (14) est un chauffage de la conduite de gaz.

3. Unité de vaporisation de produit anesthésique (2) selon l'une quelconque des revendications précédentes, **caractérisée en ce que** le chauffage (14) est un chauffage électrique.

4. Unité de vaporisation de produit anesthésique (2) selon l'une quelconque des revendications précédentes, **caractérisée en ce que** la conduite de gaz (6) est maintenue par un support (16), auquel le chauffage (14) est associé.

5. Unité de vaporisation de produit anesthésique (2) selon l'une quelconque des revendications précédentes, **caractérisée en ce que** le chauffage (14) est au moins en partie intégré dans la paroi de conduite (8).

6. Unité de vaporisation de produit anesthésique (2) selon l'une quelconque des revendications précédentes, **caractérisée en ce que** le chauffage (14) présente des lamelles (18), qui sont disposées extérieurement à proximité en contact avec la paroi de conduite de gaz (8) de la conduite de gaz (6).

7. Unité de vaporisation de produit anesthésique (2) selon l'une quelconque des revendications précédentes, **caractérisée en ce que** la mèche (10) est une mèche textile, et **en ce que** la conduite de gaz (6) est une conduite de gaz textile.

8. Unité de vaporisation de produit anesthésique (2) selon l'une quelconque des revendications précédentes, **caractérisée en ce qu'**il est prévu un capteur de température (20) pour la détection d'une température du gaz dans la conduite de gaz (6) et/ou d'une température représentant celle-ci.

9. Procédé de contrôle d'une unité de vaporisation de produit anesthésique (2) selon la revendication 8, la puissance du chauffage (14) en fonction d'un débit de gaz à travers la conduite de gaz (6), de la température détectée du gaz dans la conduite de gaz et/ou d'une concentration de produit anesthésique du gaz s'écoulant à travers la conduite de gaz (6) étant contrôlée.
